# EUROPÄISCHE PATENTANMELDUNG

(11) **EP 1 061 132 A1**
(43) Veröffentlichungstag der Anmeldung: **20.12.2000**
(21) Anmeldenummer: 00112130.0
(22) Anmeldetag: 06.06.2000
(51) Int. Cl.: C12P 7/24, C12N 9/20, C07C 15/02

(54) **Verfahren zur Herstellung von aromatischen Carbonylverbindungen aus Styrolen durch Lipasen**

(30) Priorität: 19.06.1999 DE 19928158
(71) Anmelder: HAARMANN & REIMER GMBH, D-37601 Holzminden (DE)
(72) Erfinder: Gatfield, Ian-Lucas, Dr., 37671 Höxter (DE); Hilmer, Jens-Michael, Dr., 37671 Höxter (DE)
(74) Vertreter: Mann, Volker, Dr.

(57) **Zusammenfassung**

Die vorliegende Erfindung betrifft ein neues Verfahren zur Herstellung von aromatischen Carbonylverbindungen durch oxidative Spaltung von Styrolen unter Verwendung von Lipasen und Wasserstoffperoxid oder Wasserstoffperoxid-Lieferanten in Gegenwart von Carbonsäuren oder Carbonsäureestern.

## Beschreibung

Die vorliegende Erfindung betrifft ein neues Verfahren zur Herstellung von aromatischen Carbonylverbindungen durch oxidative Spaltung von Styrolen unter Verwendung von Lipasen und Wasserstoffperoxid oder Wasserstoffperoxid-Lieferanten in Gegenwart von Carbonsäuren oder Carbonsäureestern.

Zur Herstellung von aromatischen Carbonylverbindungen, die als Geschmack- oder Riechstoffe eingesetzt werden sollen, kann die oxidative Spaltung von Styrolen genutzt werden. Für die Darstellung von Vanillin ist die oxidative Spaltung von Eugenol oder Isoeugenol mit K₂Cr₂O₇/H₂SO₄ beschrieben. Technisch wird Vanillin überwiegend durch die alkalische Hydrolyse von Lignin (Sulfitablauge aus der Zellstoffgewinnung) und oxidative Spaltung des dabei entstehenden Coniferylalkohols hergestellt (Römpp Lexikon Chemie, 10. Auflage 1999, S. 4808).

Die auf diese Weise hergestellten aromatischen Carbonylverbindungen wie beispielsweise Vanillin haben jedoch den Nachteil, daß sie im Sinne der Aromenrichtlinie 88/388/EWG nicht als natürliche Aromastoffe sondern nur als naturidentische Aromastoffe bezeichnet werden dürfen. Nur Verbindungen, die durch physikalische Prozesse (z.B. Destillation oder Extraktion) oder biotechnologische Verfahren (enzymatisch oder mikrobiell) hergestellt wurden, können als natürliche Aromastoffe bezeichnet werden.

Zur Darstellung von natürlichem Benzaldehyd wird beispielsweise Cassia-Öl mit heißem Wasserdampf behandelt.

EP-A 542 348 beschreibt ein Verfahren zur Herstellung von natürlichen Phenylaldehyden mit dem Enzym Lipoxidase. Werden als Substrate Eugenol oder Isoeugenol eingesetzt, wird durch Umsetzung mit der Lipoxidase Vanillin erhalten. Nachteilig an diesem Verfahren sind die niedrigen Umsätze von 0,3 bis 15 %.

DE-A 196 49 655 beschreibt ein Verfahren zur Darstellung von Vanillin aus Ferulasäure in Anwesenheit des Enzyms Ferulasäuredeacylase. Das Enzym ist allerdings nur für die Vanillindarstellung ausgehend von Ferulasäure einsetzbar. Andere Ausgangsstoffe kommen nicht in Frage.

US-A 5 128 253 beschreibt die Herstellung von natürlichem Vanillin aus Ferulasäure oder Eugenol durch Biotransformation. Verwendete Mikroorganismen sind *Aspergillus niger, Rhodotorula glutinis* und *Corynebacterium glutamicum*. Nachteilig sind hier vor allem die langen Inkubationszeiten von durchschnittlich 7 bis 10 Tagen.

In EP-A 405 197 wird die Herstellung von natürlichem Vanillin durch Oxidation von Eugenol oder Isoeugenol unter Verwendung von Mikroorganismen der Gattungen Serratia, Klebsiella oder Enterobacter beschrieben. Auch diese Methode erlaubt innerhalb kurzer Reaktionszeiten nur relativ geringe Ausbeuten.

Die Aufgabe der vorliegenden Erfindung war es nun, ein Verfahren zur Herstellung von aromatischen Carbonylverbindungen, die als natürliche Aroma- oder Riechstoffe eingesetzt werden können, bereitzustellen, welches einfach durchführbar ist und die gewünschten Produkte in guten Ausbeuten und in kurzen Reaktionszeiten erhältlich macht.

Es wurde ein Verfahren zur Herstellung von aromatischen Carbonylverbindungen gefunden, das dadurch gekennzeichnet ist, daß Styrole in Gegenwart von Carbonsäuren oder Carbonsäureestern durch Lipasen und Wasserstoffperoxid oder Wasserstoffperoxid-Lieferanten oxidativ gespalten werden.

Die Vorteile des erfindungsgemäßen Verfahren sind hohe Ausbeuten, einfache Durchführung und einfache Isolierung der gewünschten Produkte. Zudem können verschiedene Ausgangsstoffe mit der gleichen Lipase zu verschiedenen Produkten, die als natürliche Aromastoffe Anwendung finden, umgesetzt werden. Damit stellt das erfindungsgemäße Verfahren ein universell anwendbares Verfahren zur Darstellung von aromatischen Carbonylverbindungen aus Styrolen dar.

Lipasen werden üblicherweise zur Veresterung oder Umesterung eingesetzt. Für diese Anwendungen existieren zahlreiche Beschreibungen. Ebenfalls beschrieben ist die Verwendung von Lipasen zur enzymatischen Epoxidierung von Alkenen (WO 91/043333) und zur Darstellung von Peroxycarbonsäuren aus Carbonsäuren. Desweiteren ist die Umsetzung von Sulfiden zu Sulfoxiden (Björkling et al., *J. Chem. Soc., Chem. Commun.*, 1990, 1301-1303) und die Lipase-katalysierte Baeyer-Villiger Oxidation (Lemoult et al., *J. Chem. Soc. Perkin Trans*. 1, 1995, 89-91) beschrieben.

Im erfindungsgemäßen Verfahren bildet sich im ersten Reaktionsschritt aus der Carbonsäure bzw. dem Carbonsäureester und Wasserstoffperoxid die entsprechende Peroxycarbonsäure. Diese reagiert mit dem eingesetzten Styrol dahingehend, daß als Reaktionsprodukte aromatische Carbonylverbindungen und die entsprechende Carbonsäure erhalten werden.

Als Styrole werden bevorzugt Verbindungen der Formel (I) eingesetzt, 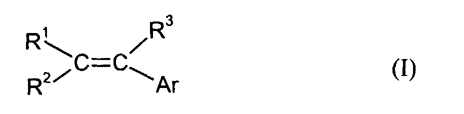 worin
- R¹, R² und R³: unabhängig voneinander Wasserstoff, geradkettiges oder verzweigtes C₁-C₂₀- Alkyl, C₃-C₈-Cycloalkyl, C₆-C₁₄-Aryl, C₇-C₁₅-ArylaIkyl, C₁-C₂₀-Alkoxy, C₁-C₂₀-Alkylamino bedeuten, wobei die zuvor genannten Kohlenwasserstoffreste gegebenenfalls ein- oder mehrfach substituiert sein können mit Hydroxy, Formyl, Oxy, C₁-C₆-Alkoxy, Carboxy, Mercapto, Sulfo, Amino, C₁-C₆-Alkylamino, Nitro oder Halogen, und
- Ar: Phenyl, gegebenenfalls ein- oder mehrfach substituiert mit geradkettigem oder verzweigtem C₁-C₆-Alkyl, C₃-C₈-Cycloalkyl, C₁-C₆-Alkoxy, Hydroxy, Oxy, Carboxy, Mercapto, Sulfo, Amino, C₁-C₆-Alkylamino, Nitro oder Halogen, bedeutet.

Besonders bevorzugt werden Styrole der Formel (I) eingesetzt,
worin
- R¹: geradkettiges oder verzweigtes C₁-C₂₀-Alkyl, C₃-C₈-Cycloalkyl, Phenyl, C₇-C₁₅-Arylalkyl, C₁-C₂₀-Alkoxy, Oxy, Formyl oder Carboxy bedeutet
- R² und R³: Wasserstoff bedeuten und
- Ar: Phenyl, gegebenenfalls ein- oder mehrfach substituiert mit geradkettigem oder verzweigtem C₁-C₆-Alkyl, C₃-C₈-Cycloalkyl, C₁-C₆-Alkoxy, Hydroxy, Formyl, Oxy, Carboxy, Mercapto, Amino, Halogen oder C₁-C₆-Alkylamino bedeutet.

Ganz besonders bevorzugt werden als Styrole Isoeugenol, Ferulasäure, Coniferylalkohol, Zimtaldehyd oder Coniferylaldeyhd eingesetzt.

Das erfindungsgemäße Verfahren wird in Gegenwart von Carbonsäuren oder Carbonsäureestern der Formel (II) durchgeführt, 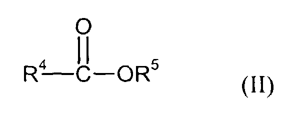 worin
- R⁴: geradkettiges oder verzweigtes C₁-C₂₀-Alkyl, C₃-C₈- Cycloalkyl, C₂-C₂₀-Alkenyl, C₅-C₈-Cycloalkenyl, C₂-C₂₀-Alkinyl, C₆-C₁₄-Aryl, C₇-C₁₅-Arylalkyl, gegebenenfalls ein- oder mehrfach substituiert mit Hydroxy, Oxy, Formyl, Mercapto, C₁-C₆-Alkoxy, C₁-C₆-Alkylamino oder Halogen, bedeutet und
- R⁵: Wasserstoff, geradkettiges oder verzweigtes C₁-C₂₀-Alkyl, C₃-C₈-Cycloalkyl, C₂-C₂₀-Alkenyl, C₅-C₈-Cycloalkenyl, C₂-C₂₀-Alkinyl, C₆-C₁₄-Aryl, C₇-C₁₅-Arylalkyl, gegebenenfalls ein- oder mehrfach substituiert mit Hydroxy, Oxy, Formyl, Mercapto, C₁-C₆-Alkoxy, C₁-C₆-Alkylamino oder Halogen, bedeutet.

Bevorzugt werden Carbonsäuren oder Carbonsäureester der Formel (II) eingesetzt, worin
- R⁴: geradkettiges oder verzweigtes, C₁-C₁₀-Alkyl, C₂-C₁₀-Alkenyl, C₂-C₁₀-Alkinyl, C₃-C₈- Cycloalkyl, C₁-C₆-Aryl oder C₇-C₁₅-Arylalkyl bedeutet und
- R⁵: Wasserstoff, geradkettiges oder verzweigtes, C₁-C₁₀-Alkyl, C₂-C₁₀-Alkenyl, C₂-C₁₀-Alkinyl, C₃-C₈- Cycloalkyl, C₁-C₆-Aryl oder C₇-C₁₅-Arylalkyl bedeutet.

Besonders bevorzugt werden als Carbonsäuren oder Carbonsäureester Essigsäure oder Essigsäureethylester eingesetzt.

Üblicherweise werden für das erfindungsgemäßen Verfahren Lipasen au*s Candida antarctica* verwendet. Bevorzugt werden Lipasen aus den *Candida antarctica-*Stämmen verwendet, die in der Deutschen Sammlung von Mikroorganismen gemäß den Vereinbarungen des Budapester Vertrags über die Hinterlegung von Mikroorganismen unter folgender Nummer hinterlegt wurden: DSM 3855, hinterlegt am 29. September 1986, DSM 3908 und DSM 3909, hinterlegt am 8. Dezember 1986.

Die Lipasen sind nach einem in WO 88/02775 beschrieben Verfahren aus den jeweiligen Mikroorganismen erhältlich:
Nach Kultivierung des entsprechenden Stamms in einem geeigneten Nährmedium unter aeroben Bedingungen können flüssige Enzym-Konzentrate nach Entfernen von unlöslichem Material (z.B. durch Filtration oder Zentrifugation) durch Evaporation oder Umkehrosmose gewonnen werden. Feste Enzympräparationen können durch Ausfällung aus dem löslichen Konzentrat durch Zugabe von Salzen oder Ethanol erhalten werden.

Es ist bekannt, daß Lipasen auch durch rekombinante DNA-Techniken gewonnen werden können (EP 238 023). Dabei wird das für die Lipase codierende Gen von einem ausgewählten Stamm durch dem Fachmann bekannte Methoden in einen Empfängerorganismus transferiert. Dieser Empfängerorganismus produziert die Lipase.

In einer besonders bevorzugten Ausführung werden rekombinante Lipasen, die auf einem Trägermaterial immobilisiert sind, verwendet. Geeignete Trägermaterialien sind beispielsweise Kunststoffe wie Polypropylen, Polystyrol, Polyvinylchlorid, Polyurethan, Polyacryl, Latex, Nylon oder Teflon, Polysaccaride wie Agarose oder Dextran, Ionenaustauscherharze (sowohl kationische wie anionische), Silicon-Polymere wie beispielsweise Siloxane oder Silicate, beispielsweise Glas. Immobilisierungstechniken für Enzyme sind dem Fachmann bekannt (K. Mosbach, 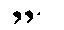 Immobilized Enzymes", *Methods in Enzymology* 44, Academic Press, New York, 1976) und beinhalten cross-linking, Adsorption oder kovalente Bindung an das Trägermaterial.

Lipasen aus *Candida antarctica* werden auch kommerziell vertrieben, beispielsweise Chirazyme L-2, c-f, Lyo (Vertreiber: Roche Diagnostics GmbH, No. 1663917103), Chirazyme L-2, c-f, C2 Lyo (Vertreiber: Roche Diagnostics GmbH, No. 1816969103) oder Novozym 435 (Novo Nordisk).

Die Konzentration an Wasserstoffperoxid, die für das erfindungsgemäße Verfahren benötigt wird, liegt im Bereich von 0,05 Vol.% bis 10 Vol.%. Bevorzugt arbeitet man bei einer Wasserstoffperoxid-Konzentration von 0,05 Vol.% bis 5 Vol.%, besonders bevorzugt wird das erfindungsgemäße Verfahren bei einer Wasserstoffperoxid-Konzentration von 0,05 Vol.% bis 0,5 Vol.% durchgeführt.

Um die für das erfindungsgemäße Verfahren benötigte Wasserstoffperoxid-Konzentration in der Reaktionsmischung einzustellen, kann wässrige Wasserstoffperoxid-Lösung verwendet werden. Bevorzugt wird 30 % bis 60 % (w/v) wässrige Wasserstoffperoxid-Lösung zur Einstellung der im erfindungsgemäßen Verfahren benötigten Wasserstoffperoxid-Konzentration verwendet.

Der Zusatz von Wasserstoffperoxid-Lösung kann zu Beginn der Reaktion oder aber tropfenweise während des Reaktionsverlaufs erfolgen.

Die Wasserstoffperoxid-Konzentration in der Reaktionsmischung kann auch durch Wasserstoffperoxid-Lieferanten, die *in situ* Wasserstoffperoxid bilden, eingestellt werden. Wasserstoffperoxid-Lieferanten sind beispielsweise Percarbonate oder Perborate oder das Enzym Glucoseoxidase in Verbindung mit Glucose.

Bevorzugt wird das Enzym Glucoseoxidase in Verbindung mit Glucose als Wasserstoffperoxid-Lieferant im erfindungsgemäßen Verfahren eingesetzt.

Bei der Glucoseoxidase handelt es sich bevorzugt um Glucoseoxidase aus *Aspergillus niger*, welche in ihrer nativen Form oder immobilisiert im erfindungsgemäßen Verfahren eingesetzt werden kann. In einer bevorzugten Form des erfindungsgemäßen Verfahrens wird die Glucoseoxidase in immobilisierter Form eingesetzt.

Üblicherweise können als Lösungsmittel für das erfindungsgemäße Verfahren Wasser, wässrige Puffer und organische Lösungsmittel eingesetzt werden. Als organische Lösungsmittel werden bevorzugt Hexan, Cyclohexan, Heptan, Cycloheptan, Toluol, Dichlormethan, Acetonitril, Dimethylformamid, Dioxan, Tetrahydrofuran oder Ethanol verwendet. Als wässrige Puffer werden bevorzugt Phosphat- oder Acetatpuffer verwendet.

In einer bevorzugten Form des erfindungsgemäßen Verfahrens werden entweder die zu oxidierenden Styrole oder die Carbonsäuren oder Carbonsäureester im Überschuß als Lösungsmittel eingesetzt. Werden die Carbonsäuren oder Carbonsäureester im erfindungsgemäßen Verfahren als Lösungsmittel eingesetzt, so wird bevorzugt Essigsäure oder Essigsäureethylester verwendet.

Das erfindungsgemäße Verfahren kann mit Überschüssen, äquimolaren Mengen oder aber mit einem Unterschuß an Carbonsäure beziehungsweise Carbonsäureester bezogen auf das eingesetzte Styrol durchgeführt werden. Wird die Carbonsäure oder der Carbonsäureester als Lösungsmittel eingesetzt, so liegen diese Verbindungen im Überschuß bezogen auf das eingesetzte Styrol vor. Wird die Reaktion in einem anderen Lösungsmittel durchgeführt, so kann die Carbonsäure oder der Carbonsäureester entweder äquimolar bezogen auf das zu oxidierende Styrol oder aber im Unterschuß eingesetzt werden, da sich diese Verbindungen, die während des Reaktionsverlaufs intermediär zu Peroxycarbonsäuren oxidiert werden, wieder zu den entsprechenden Carbonsäuren regenerieren.

Üblicherweise liegt die Temperatur, bei der das erfindungsgemäße Verfahren durchgeführt wird, in einem Bereich von 1°C bis 95°C, bevorzugt zwischen 10°C und 70°C, besonders bevorzugt zwischen 15°C und 50°C.

### Beispiele

### Beispiel 1

In einem Erlenmeyerkolben wurden 50 ml Essigsäureethylester, 0,82 g (5 mmol) Isoeugenol, 8 ml 30% (w/v) wässrige Wasserstoffperoxid-Lösung und 50 mg (122 U) Lipase Chirazyme L-2, c-f, C2 lyo (Roche Diagnostics GmbH) gemischt. Die Mischung wurde auf einem automatischen Schüttler für 24 h bei 25°C geschüttelt. Zur Bestimmung des Anteils an gewünschtem Reaktionsprodukt Vanillin in der Mischung wurden vom Überstand nach 24 h Proben genommen (Analysedaten Tabelle 1, Zeile 1). Die Aufarbeitung erfolgte durch Zusatz von Kaliumpermanganat, wodurch überschüssiges Wasserstoffperoxid entfernt wurde und Trocknen der Proben über Natriumsulfat (Analysedaten Tabelle 1, Zeile 2). Die Analyse der Proben erfolgte gaschromatographisch auf einer OV-351-Säule. Die Ergebnisse der Analysen sind in Tabelle 1 wiedergegeben, wobei die Prozentangaben durch die Fläche der Peaks im Gaschromatogramm berechnet wurden. Die Menge an Essigsäure wurde nicht mitgerechnet.

**Tabelle 1**

| Reaktionsdauer | Isoeugenol [GC %] | Vanillin [GC %] |
|---|---|---|
| 24 h | 0,7 | 45,7 |
| 24 h, nach Aufarbeitung | 0,7 | 30,4 |

### Beispiel 2

In einem Erlenmeyerkolben wurden 50 ml Essigsäureethylester, 3,56 g (20 mmol) Coniferylaldehyd, 10 ml 30% (w/v) wässrige Wasserstoffperoxid-Lösung und 2,5 g (6125 U) Lipase Chirazyme L-2, c-f, C2 lyo (Roche Diagnostics GmbH) zusammengegeben. Die Mischung wurde auf einem automatischen Schüttler für 48 h bei 25°C geschüttelt. Zur Bestimmung des Anteils an gewünschtem Reaktionsprodukt Vanillin in der Mischung wurden vom Überstand nach 24 h bzw. 48 h Proben genommen, über Sterilfilter filtriert und über Natriumsulfat getrocknet. Zur Aufarbeitung wurde die Mischung nach 48 h mit Kaliumpermanganat behandelt und der entstandene Braunstein abfiltriert. Danach wurde die Essigsäure mit Natriumcarbonat neutralisiert und die Lösung wurde einmal mit Eiswasser gewaschen. Die organische Phase wurde über Natriumsulfat getrocknet und am Rotationsverdampfer eingeengt. Die Analysedaten der Proben nach 24 h bzw. 48 h und die der aufgearbeiteten Probe finden sich in Tabelle 2. Die Analyse der Proben erfolgte gaschromatographisch auf einer OV-351-Säule, wobei die Prozentangaben durch die Fläche der Peaks im Gaschromatogramm berechnet wurden. Die Menge an Essigsäure wurde nicht mitgerechnet.

**Tabelle 2**

| Reaktionsdauer | Vanillin [GC %] |
|---|---|
| 24h | 82,2 |
| 48h | 83,7 |
| 48 h, nach Aufarbeitung | 83,1 |

### Beispiel 3

In einem Erlenmeyerkolben wurden 50 ml Essigsäureethylester, 0,6 g (4,5 mmol) Zimtaldehyd, 10 ml 30% (w/v) wässrige Wasserstoffperoxid-Lösung und 150 mg (360 U) Lipase Chirazyme L-2, c-f, C2 lyo (Roche Diagnostics GmbH) zusammengegeben. Die Mischung wurde auf einem automatischen Schüttler für 24 h bei 25°C geschüttelt. Zur Bestimmung des Anteils an den gewünschten Reaktionsprodukten Benzaldehyd und Phenylacetaldehyd in der Mischung wurden vom Überstand Proben genommen, über Sterilfilter filtriert und über Natriumsulfat getrocknet. Die Analyse der Proben erfolgte gaschromatographisch auf einer OV-351-Säule, wobei die Prozentangaben durch die Fläche der Peaks im Gaschromatogramm berechnet wurden. Als Vergleich wurde die Reaktion wie oben, allerdings ohne den Zusatz von Lipase durchgeführt. Die Resultate beider Versuche finden sich in Tabelle 3, wobei die Menge an Essigsäure nicht mitgerechnet wurde.

**Tabelle 3**

| | Benzaldehyd | Phenylacetaldehyd | Benzoesäure | Zimtsäure | Zimtaldehyd |
|---|---|---|---|---|---|
| mit Enzym | 29,1% | 6,2% | 15,9% | 19,4% | 13,0% |
| ohne Enzym | 0% | 0% | 0,3% | 0,6% | 94,8% |

## Patentansprüche

1. Verfahren zur Herstellung von aromatischen Carbonylverbindungen, dadurch gekennzeichnet, daß Styrole in Gegenwart von Carbonsäuren oder Carbonsäureestern durch Lipasen und Wasserstoffperoxid oder einem Wasserstoffperoxid-Lieferanten oxidativ gespalten werden.

2. Verfahren gemäß Anspruch 1, dadurch gekennzeichnet, daß Styrole der Formel (I) eingesetzt werden, 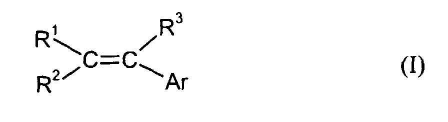 worin
R¹, R² und R³ unabhängig voneinander Wasserstoff, geradkettiges oder verzweigtes C₁-C₂₀- Alkyl, C₃-C₈-Cycloalkyl, C₆-C₁₄-Aryl, C₇-C₁₅-Arylalkyl, C₁-C₂₀-Alkoxy, C₁-C₂₀-Alkylamino bedeuten, wobei die zuvor genannten Kohlenwasserstoffreste gegebenenfalls ein- oder mehrfach substituiert sein können mit Hydroxy, Formyl, Oxy, C₁-C₆-Alkoxy, Carboxy, Mercapto, Sulfo, Amino, C₁-C₆-Alkylamino oder Nitro oder Halogen, und
Ar Phenyl, gegebenenfalls ein- oder mehrfach substituiert mit geradkettigem oder verzweigtem C₁-C₆-Alkyl, C₃-C₈-Cycloalkyl, C₁-C₆-Alkoxy, Hydroxy, Oxy, Carboxy, Mercapto, Sulfo, Amino, C₁-C₆-Alkylamino, Nitro oder Halogen, bedeutet.

3. Verfahren gemäß einem oder mehreren der vorangegangenen Ansprüche 1 bis 2, dadurch gekennzeichnet, daß die Styrole der Formel (I) entsprechen
worin
R¹ geradkettiges oder verzweigtes C₁-C₂₀-Alkyl, C₃-C₈-Cycloalkyl, Phenyl, C₇-C₁₅-Arylalkyl, C₁-C₂₀-Alkoxy, Oxy, Formyl oder Carboxy bedeutet
R² und R³ Wasserstoff bedeuten und
Ar Phenyl, gegebenenfalls ein- oder mehrfach substituiert mit geradkettigem oder verzweigtem C₁-C₆-Alkyl, C₃-C₈-Cycloalkyl, C₁-C₆-Alkoxy, Hydroxy, Formyl, Oxy, Carboxy, Mercapto, Amino, Halogen oder C₁-C₆-Alkylamino, bedeutet.

4. Verfahren gemäß einem oder mehreren der vorangegangenen Ansprüche 1 bis 3, dadurch gekennzeichnet, daß als Styrole Isoeugenol, Ferulasäure, Coniferylaldehyd oder Zimtaldehyd eingesetzt werden.

5. Verfahren gemäß einem oder mehreren der vorangegangenen Ansprüche 1 bis 4, dadurch gekennzeichnet, daß Carbonsäuren oder Carbonsäureester der Formel (II) eingesetzt werden, 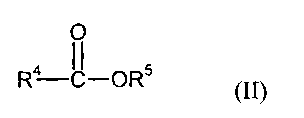 worin
R⁴ geradkettiges oder verzweigtes C₁-C₂₀-Alkyl, C₃-C₈- Cycloalkyl, C₂-C₂₀-Alkenyl, C₅-C₈-Cycloalkenyl, C₂-C₂₀-Alkinyl, C₆-C₁₄-Aryl, C₇-C₁₅-Arylalkyl, gegebenenfalls ein- oder mehrfach substituiert mit Hydroxy, Oxy, Formyl, Mercapto, C₁-C₆-Alkoxy, C₁-C₆-Alkylamino oder Halogen, bedeutet und
R⁵ Wasserstoff, geradkettiges oder verzweigtes C₁-C₂₀-Alkyl, C₃-C₈-Cycloalkyl, C₂-C₂₀-Alkenyl, C₅-C₈-Cycloalkenyl, C₂-C₂₀-Alkinyl, C₆-C₁₄-Aryl, C₇-C₁₅-Arylalkyl, gegebenenfalls ein- oder mehrfach substituiert mit Hydroxy, Oxy, Formyl, Mercapto, C₁-C₆-Alkoxy, C₁-C₆-Alkylamino oder Halogen, bedeutet.

6. Verfahren gemäß einem oder mehreren der vorangegangenen Ansprüche 1 bis 5, dadurch gekennzeichnet, daß die Lipase aus *Candida antarctica* stammt.

7. Verfahren gemäß einem oder mehreren der Ansprüche 1 bis 6, dadurch gekennzeichnet, daß die Wasserstoffperoxid-Konzentration in der Reaktionsmischung zwischen 0,05 Vol.% und 10 Vol.%, bevorzugt zwischen 0,05 Vol.% und 5 Vol.% und besonders bevorzugt zwischen 0,05 Vol.% und 0,5 Vol.% liegt.

8. Verfahren gemäß einem oder mehreren der vorangegangenen Ansprüche 1 bis 7, dadurch gekennzeichnet, daß die Wasserstoffperoxid-Konzentration in der Reaktionsmischung *in situ* durch den Wasserstoffperoxid-Lieferanten Glucoseoxidase in Verbindung mit Glucose gebildet wird.

9. Verfahren gemäß Anspruch 8, dadurch gekennzeichnet, daß die Glucoseoxidase aus *Aspergillus niger* stammt.

10. Verfahren gemäß einem oder mehreren der vorangegangenen Ansprüche 1 bis 9, dadurch gekennzeichnet, daß als Lösungsmittel ein Überschuß an Styrol oder Carbonsäure oder Carbonsäureester eingesetzt wird.

11. Verfahren gemäß einem oder mehreren der vorangegangenen Ansprüche 1 bis 10, dadurch gekennzeichnet, daß die Reaktion in einem Temperaturbereich von 1°C bis 95°C durchgeführt wird.

12. Aromatische Carbonylverbindungen, herstellbar durch Umsetzung von Styrolen mit Lipasen und Wasserstoffperoxid oder Wasserstoffperoxid-Lieferanten in Gegenwart von Carbonsäuren oder Carbonsäureestern.
